# EUROPEAN PATENT APPLICATION

(11) **EP 4 442 806 A1**
(43) Date of publication of application: **09.10.2024**
(21) Application number: 23869687.6
(22) Date of filing: 31.05.2023
(51) Int. Cl.: C12N 1/20, A61K 8/99, A61K 35/74, A61Q 19/08, A23L 33/21, A23L 33/135, A23L 33/105, A23L 33/125, A23K 10/18, A61P 3/06, A61P 35/00, C12R 1/01

(54) **AKKERMANSIA GUANGXIENSIS CAPABLE OF RESISTING OXIDATION, REDUCING FAT AND INHIBITING TUMOR GROWTH, AND PRODUCT AND USE THEREOF**

(30) Priority: 30.03.2023 CN 202310332284
(71) Applicant: Aiage Life Science Corporation Ltd., Nanning, Guangxi 530200 (CN)
(72) Inventor: XIAO, Guilong, Nanning, Guangxi 530200 (CN); LUO, Weifei, Nanning, Guangxi 530200 (CN); LU, Zhilong, Nanning, Guangxi 530200 (CN); WEI, Xinli, Nanning, Guangxi 530200 (CN); MENG, Lili, Nanning, Guangxi 530200 (CN); YIN, Meng, Nanning, Guangxi 530200 (CN); LU, Enqiu, Nanning, Guangxi 530200 (CN); PANG, Shifu, Nanning, Guangxi 530200 (CN)
(74) Representative: Gassner, Birgitta
(86) International application number: PCT/CN2023/097509
(87) International publication number: WO 2024/066434

(57) **Abstract**

Disclosed in the invention are an *Akkermansia* and a product and use thereof. The *Akkermansia* is *Akkermansia guangxiensis* N21116 and N21169, and the accession numbers are respectively: GDMCC No: 62888 and GDMCC No: 62889. *Akkermansia guangxiensis* N21116 and N21169 are new species of *Akkermansia,* have resistance to human intestinal fluid, and have the effects of resisting oxidation, inhibiting tumor cell growth, and losing weight and reducing fat.

## Description

### TECHNICAL FIELD

The present invention relates to the technical field of microorganisms, in particular to *Akkermansia guangxiensis* capable of resisting oxidation, reducing fat and inhibiting tumor growth, and a product and use thereof.

### BACKGROUND

Population aging is one of the most valued and important social issues in the 21st century. According to relevant surveys, it is expected that the proportion of the aging population (over 65 years old) will reach 16% by 2050. Countries around the world are facing the problem of aging, including China. Currently, the number of people aged 65 and above exceeds 200 million, accounting for 14.2% of the total population. It is expected that China's elderly population aged 60 and above will exceed 400 million by around 2035.

The prominence of the aging problem has driven the world to pay increasing attention to health and aging issues. At the same time, with the improvement of the world's economic level, the pursuit of a longer healthy life span has become a common consensus among the public. At present, academic circles generally believe that the longevity mechanism is a complex system, and it is inseparable from antioxidant mechanisms, chronic inflammation mechanisms, metabolic disorders and other mechanisms. Therefore, the development of drugs or supplements with antioxidant properties, inhibition of inflammation, and improvement of metabolic disorders has become an important way to achieve health and longevity.

Guangxi is one of the provinces with the most dense distribution of longevity counties in China, and the study of its longevity elderly and longevity phenomenon is a current hotspot. In particular, studying the characteristics of the intestinal flora of long-lived elderly people and its relationship with elderly chronic diseases has become a current research hotspot. The intestinal flora is widely linked to various chronic diseases, and emerging evidences have shown that the intestinal flora plays an important and irreplaceable role in resisting oxidation, inhibiting inflammation and improving metabolism. However, currently available probiotics with the above anti-aging functions are relatively rare, and therefore, the development of new probiotics with related functions derived from long-lived elderly people is of great significance for the development of human anti-aging products.

### SUMMARY

The present invention aims to solve at least one of the above technical problems existing in the prior art. To this end, the present invention provides *Akkermansia guangxiensis* capable of resisting oxidation, reducing fat and inhibiting tumor growth, and a product containing the *Akkermansia guangxiensis* and use thereof. Two strains of *Akkermansia muciniphila* in the present invention are isolated from fecal samples of centenarians in Guangxi, and it has been found that they have stronger antioxidant capacity, fat-reducing ability and stronger inhibition of tumor cell proliferation than other *Akkermansia muciniphila,* with extremely high development value and practical value, thus providing new ideas for the development and application of antioxidant, tumor-inhibiting and fat-reducing products.

In first aspect of the present invention, *Akkermansia muciniphila* is provided, which is *Akkermansia guangxiensis* N21116, with a taxonomic name of *Akkermansia* sp., deposited in the Guangdong Microbial Culture Collection Center on December 2, 2022, with an accession number of GDMCC No: 62888.

In second aspect of the present invention, *Akkermansia muciniphila* is provided, which is *Akkermansia guangxiensis* N21169, with a taxonomic name of *Akkermansia* sp., deposited in the Guangdong Microbial Culture Collection Center on December 2, 2022, with an accession number of GDMCC No: 62889.

In some embodiments of the present invention, the *Akkermansia guangxiensis* N21116 and N21169 are both screened and isolated from fecal samples of centenarians in Guangxi.

In some embodiments of the present invention, colony morphologies of the *Akkermansia guangxiensis* N21116 and N21169 are both round, convex, smooth, neatly-edged and grey white colonies.

In some embodiments of the present invention, a 16S sequence of the *Akkermansia guangxiensis* N21116 is as shown in SEQ ID NO: 5.

In some embodiments of the present invention, a 16S sequence of the *Akkermansia guangxiensis* N21169 is as shown in SEQ ID NO: 6.

In some embodiments of the present invention, the screening and isolation method of the *Akkermansia guangxiensis* N21116 and N21169 comprises: taking fecal samples of centenarians in Guangxi and placing the samples into a solid culture medium with mucin, culturing for 3-6 days under anaerobic conditions, picking out the colonies in the culture medium that are round, convex, smooth, neatly-edged and grey white, and obtaining the *Akkermansia guangxiensis* N21116 and N21169 after a polymerase chain reaction (PCR) amplification identification.

In the present invention, by testing antioxidant levels (hydroxyl radical scavenging rate, peroxide anion scavenging rate and superoxide dismutase (SOD) activity), the *Akkermansia guangxiensis* N21116 and N21169 obtained through the above manual screening and isolation are determined to have stronger antioxidant capacities than strains of the same genus, thus products prepared therefrom show better antioxidant effects.

In some embodiments of the present invention, a culture time of the *Akkermansia guangxiensis* N21116 and N21169 is 40-72 h.

In some embodiments of the present invention, a culture temperature of the *Akkermansia guangxiensis* N21116 and N21169 is 30°C-37°C, and a rotation speed is 150 rpm-220 rpm.

In some embodiments of the present invention, *Akkermansia muciniphila* specific-amplification primers are used for the PCR amplification identification.

In some embodiments of the present invention, the *Akkermansia muciniphila* specific-amplification primers are as shown in SEQ ID NOs: 1 and 2.

In some embodiments of the present invention, an amplification system for the PCR amplification identification is as shown in Table 1.

In some embodiments of the present invention, thermal cycle parameters for the PCR amplification identification are set as: pre-denaturation for 3 min at 95°C; denaturation for 40 s at 95°C, annealing for 30 s at 56°C, extension for 1 min at 72°C, 35 cycles; and finally extension for 10 min at 72°C.

Of course, those skilled in the art can also reasonably adjust the content and concentration in the PCR amplification system as well as the amplification procedure according to actual needs, so as to achieve the identification of the *Akkermansia muciniphila.*

In third aspect of the present invention, a product containing the *Akkermansia muciniphila* according to the first and/or second aspect of the present invention is provided, and the product includes food, food additives, feeds, feed additives, medicines and cosmetics.

In some embodiments of the present invention, the product further comprises a product containing a microbial agent, a bacterial solution or a culture of the *Akkermansia muciniphila* described in the first and/or second aspect of the present invention.

In some embodiments of the present invention, a mass fraction of the *Akkermansia guangxiensis* in the product is greater than or equal to 5%.

In some embodiments of the present invention, the mass fraction of the *Akkermansia guangxiensis* in the product is greater than or equal to 8%.

In some embodiments of the present invention, the mass fraction of the *Akkermansia guangxiensis* in the product is greater than or equal to 10%.

In some embodiments of the present invention, the mass fraction of the *Akkermansia guangxiensis* in the product is 5%-15%.

In some embodiments of the present invention, the product further comprises other excipients.

In some embodiments of the present invention, the excipients include pharmaceutically acceptable adjuvants, food additives, and cosmetic accessories.

In some embodiments of the present invention, the pharmaceutically acceptable adjuvants include but are not limited to diluents (such as starch, dextrin, sucrose, lactose, mannitol and so on), absorbents (such as calcium sulfate, calcium hydrogen phosphate and so on), wetting agents (such as ethanol), binders (such as hypromellose, povidone and so on), disintegrants (such as sodium hydroxymethyl starch, crospovidone and so on), lubricants (such as talc, hydrogenated vegetable oil, polyethylene glycol and so on), colorants (such as titanium dioxide, methylene blue and so on), coating materials, solvents, pH regulators, antibacterial agents (such as sodium sulfite, sodium thiosulfate and so on), isoosmotic adjusting agents (such as glucose, sodium chloride and so on), chelating agents (such as disodium EDTA).

In some embodiments of the present invention, the food additives include but are not limited to colorants, enzyme preparations, thickeners, leavening agents, and sweeteners.

In some embodiments of the present invention, the cosmetic accessories include but are not limited to essences, pigments, preservatives, and antioxidants.

In some embodiments of the present invention, the *Akkermansia guangxiensis* in the product has forms of freeze-dried powder, bacterial solution, and granular inoculant. Of course, it is understood that those skilled in the art can also reasonably select appropriate forms of *Akkermansia muciniphila* for use according to actual use needs and existing processing techniques, including but not be limited to the above-mentioned freeze-dried powder, bacterial solution and granular inoculant.

In the present invention, the term "freeze-dried powder" refers freezing water in the medicinal liquid or bacterial solution in advance by a vacuum freeze-drying method with a freeze dryer, and then sublimating the frozen water in the medicinal liquid or bacterial solution in a vacuum sterile environment, thus obtaining a freeze-dried product.

In the present invention, the term "granular inoculant" refers to an inoculant used in order to prevent the powder inoculant from directly contacting the bactericide or chemical fertilizer during use, resulting in a reduction in the effect, and the granular inoculant usually refers to an inoculant type obtained by mixing the bacterial solution with granular carriers (such as biochar, vermiculite and so on).

In some embodiments of the present invention, an effective bacterial activity of the *Akkermansia guangxiensis* in the product is 10⁷ cfu/mL-10⁹ cfu/mL.

Of course, those skilled in the art can reasonably adjust its activity according to the actual form of the *Akkermansia guangxiensis* in the product, thereby achieving stable technical effects.

In fourth aspect of the present invention, a food additive containing the *Akkermansia muciniphila* according to the first and/or second aspect of the present invention is provided, and the food additive includes freeze-dried powder of the *Akkermansia muciniphila* in the first and/or second aspect, plant extracts, maltodextrin and dietary fiber.

In some embodiments of the present invention, the plant extracts include but are not limited to plant essential oils, saponins, alkaloids, polysaccharides, polyphenols, and flavonoids.

In some embodiments of the present invention, the plant extracts are tea polyphenol and bayberry anthocyanin extracts.

In some embodiments of the present invention, the dietary fiber includes soluble dietary fiber and insoluble dietary fiber.

In some embodiments of the present invention, the dietary fiber is soluble dietary fiber.

In some embodiments of the present invention, in terms of mass percentage, the food additives include: 5-15% *Akkermansia muciniphila* freeze-dried powder in the first and/or second aspect of the present invention, 15-40% plant extracts, 40-60% starch hydrolyzed polysaccharide and 5-15% dietary fiber.

In some embodiments of the present invention, in terms of mass percentage, the food additives include: 10% *Akkermansia guangxiensis* N21116 or N21169 freeze-dried powder, 15% tea polyphenol, 15% bayberry anthocyanin extract, 50% maltodextrin and 10% soluble dietary fiber.

In some embodiments of the present invention, a preparation method of the food additive includes: obtaining the food additive after completely mixing the *Akkermansia muciniphila* freeze-dried powder in the first and/or second aspect of the present invention, the plant extracts, the maltodextrin and the dietary fiber.

In fifth aspect of the present invention, use of the *Akkermansia muciniphila* in the first and/or second aspect of the present invention in the preparation of food, food additives, feeds, feed additives, medicines and cosmetics is provided.

In the present invention, a safety assessment shows that the two strains of *Akkermansia muciniphila* do not contain pathogenic genes and virulence genes, and an imitative gastrointestinal environment resistance experiment shows that both strains can tolerate the gastrointestinal environment. Based on the above characteristics, it can be concluded that the two strains are probiotics capable of resisting oxidation, reducing fat and inhibiting tumor proliferation.

In some embodiments of the present invention, the food, the food additives, the feeds, the feed additives, the medicines and the cosmetics have at least one of the following functions (1) to (3):
(1) resisting oxidation;
(2) reducing fat; and
(3) inhibiting tumor proliferation.

In the present invention, a nematode (*Caenorhabditis elegans*) screening platform is used to verify the fat-lowering effect. The *Caenorhabditis elegans* is simple in structure, transparent in body, easy to observe, short in development cycle, easy to cultivate artificially, and highly conserved in signaling pathway, resulting in a high accuracy in verifying the effect of *Caenorhabditis elegans* model and a low experimental cost. In the present invention, through a fat-reducing test based on the *Caenorhabditis elegans,* it is effectively proved that the *Akkermansia guangxiensis* N21116 and N21169 have weight-losing and fat-reducing effects.

In the present invention, by testing antioxidant levels (hydroxyl radical scavenging rate, peroxide anion scavenging rate and superoxide dismutase (SOD) activity), the two strains of *Akkermansia guangxiensis* N21116 and N21169 are determined to have effects that exceed the antioxidant capacity of strains of the same genus and have good antioxidant capacities.

In some embodiments of the present invention, the tumor is gastrointestinal tumor.

In some embodiments of the present invention, the gastrointestinal tumor includes colon cancer.

In the present invention, by testing the effects of the two strains of *Akkermansia guangxiensis* N21116 and N21169 on the growth of human colon cancer cell line HCT116, it is confirmed that both strains can significantly inhibit the growth of human colon cancer cells and have the potential to inhibit tumor proliferation.

In sixth aspect of the present invention, a method for preventing and/or treating tumors is provided, comprising: administering a therapeutically effective amount of *Akkermansia muciniphila* to a tumor patient, wherein the *Akkermansia muciniphila* is the *Akkermansia guangxiensis* N21116 and/or *Akkermansia guangxiensis* N21169.

In some embodiments of the present invention, the tumor is gastrointestinal tumor.

In some embodiments of the invention, the gastrointestinal tumor includes colon cancer.

The beneficial effects of the present invention are as follows:
1. In the present invention, a new species of *Akkermansia muciniphila* has been discovered for the first time, which has excellent resistance to an artificial intestinal fluid, and has excellent effects of resisting oxidation, inhibiting tumor cell proliferation and reducing fat, thus effectively developing related products based on this strain, with an extremely high practical value.
2. Compared with the existing *Akkermansia muciniphila,* the new species of *Akkermansia muciniphila* in the present invention have stronger colonization ability, effects of resisting oxidation, inhibiting tumor cell proliferation and reducing fat, and extremely high application value and commercial development value.

### BRIEF DESCRIPTION OF DRAWINGS

FIG. 1 is an evolutionary tree o*f Akkermansia guangxiensis* N21116 and N21169 in examples of the present invention.
FIG. 2 is an average nucleotide identity (ANI) analysis of *Akkermansia guangxiensis* in examples of the present invention, where Agl[T] represents *Akkermansia glycaniphila* Pyt[T]; Amu[T] represents *Akkermansia muciniphila* ATCC BAA-835[T]; AmuYL44 represents *Akkermansia muciniphila* YL44; Amu22959 represents *Akkermansia muciniphila* DSM 22959[T]; AguN21116 represents *Akkermansia guangxiensis* N21116; and AguN21169 represents *Akkermansia guangxiensis* N21169.
FIG. 3 shows a tolerance result of *Akkermansia guangxiensis* N21116 and N21169 to an artificial intestinal fluid in examples of the present invention, where AmucT represents a standard strain of *Akkermansia muciniphila* ATCC BAA-835.
FIG. 4 shows an antioxidant level test result of *Akkermansia guangxiensis* N21116 and N21169 in examples of the present invention, where a is a hydroxyl radical scavenging activity test result; b is a peroxide anion scavenging activity test result; c is a superoxide dismutase SOD activity test result; AmucT represents a standard strain of *Akkermansia muciniphila* ATCC BAA-835; and A21028 represents *Akkermansia muciniphila* A21028.
FIG. 5 shows an inhibitory effect of *Akkermansia guangxiensis* N21116 and N21169 on fat accumulation in nematodes in examples of the present invention, where AmucT represents a standard strain of *Akkermansia muciniphila* ATCC BAA-835; and A21028 represents *Akkermansia muciniphila* A21028.
FIG. 6 shows a fat staining result of nematodes fed with different *Akkermansia muciniphila,* where AmucT represents a standard strain of *Akkermansia muciniphila* ATCC BAA-835; and A21028 represents *Akkermansia muciniphila* A21028.
FIG. 7 shows an inhibitory effect of *Akkermansia guangxiensis* N21116 and N21169 on a human colon cancer cell line HCT116 in examples of the present invention, where AmucT represents a standard strain of *Akkermansia muciniphila* ATCC BAA-835.

### DETAILED DESCRIPTION

The content of the present invention will be further described in detail below through specific examples. Unless otherwise specified, the raw materials, reagents or apparatus used in the examples and comparative examples can be obtained from conventional commercial sources, or can be obtained through existing technical methods. Unless otherwise stated, assays or test methods are routine in the art.

In the following examples, the components and content of the solid culture medium with mucin used are: based on the final concentration, 38 g/L of brain heart infusion (BHI), 4 g/L of mucin, and 15 g/L of agar; and obtaining the solid culture medium with mucin after mixing the above components evenly in proportion and then autoclaving for 20 min at 115°C.

In the following examples, the components and contents of the synthetic liquid culture medium used are: based on the final concentration, 38 g/L of BHI, 16 g/L of soy peptone, 4 g/L of threonine, 25 mM of glucose, and 25 mM of N-acetylglucosamine; and obtaining the synthetic liquid culture medium after mixing the above components evenly in proportion and then autoclaving for 20 min at 115°C.

### Isolation and identification of Akkermansia guangxiensis N21116 and N21169

The isolation and purification steps of *Akkermansia guangxiensis* in this example were as follows:
An appropriate amount of fecal samples from two centenarians in Guangxi were taken, diluted with phosphate buffer saline (PBS), evenly spread into the solid culture medium with mucin , and placed in an anaerobic workstation. The fecal samples were cultured for two days at 37°C under gas conditions of 90% (v/v) N₂ and 10% CO₂, and after the end of the culture, round, convex , smooth and neatly-edged and grey white colonies in plate were picked out for identification with polymerase chain reaction (PCR) amplification technique.

The PCR identification method of *Akkermansia guangxiensis* in this example was as follows:
a sterile toothpick was used to pick colonies that met the above morphological characteristics of *Akkermansia muciniphila,* the colonies were transferred to a 2× PCR premixed solution, and forward and reverse specific primers and sterile water were added in sequence (the specific PCR amplification system was as shown in Table 1). Thermal cycle parameters were set as: pre-denaturation for 3 min at 95°C; denaturation for 40 s at 95°C, annealing for 30 s at 56°C, extension for 1 min at 72°C, 35 cycles; final extension for 10 min at 72°C.

**Table 1 PCR amplification system of Akkermansia muciniphila**

| Component | Content |
|---|---|
| 10 µM forward primer F | 1 µL |
| 10 µM reverse primer R | 1 µL |
| PCR amplification solution | 12.5 µL |
| DNA template (colony) | 1 µL |
| ddH₂O | Make up to 25 µL |

The *Akkermansia muciniphila* specific amplification primers used for identification were:
Forward primer F: 5'-CAGCACGTGAAGGTGGGGAC-3' (SEQ ID NO: 1);
Reverse primer R: 5'- CCTTGCGGTTGGCTTCAGAT-3' (SEQ ID NO: 2).

The amplification product was detected using agarose gel electrophoresis. When the length of the amplified fragment met the expectation, the isolated strain could be preliminarily determined to be a suspected *Akkermansia* sp. strain.

The strain identified as suspected *Akkermansia* sp. by PCR amplification was further subjected to 16S rRNA molecular identification.

The 16S rRNA molecular identification system is the same as Table 1, with thermal cycle parameters of pre-denaturation for 2 min at 95°C, denaturation for 40 s at 95°C, annealing for 30 s at 56°C, extension for 90 s at 72°C, 30 cycles, and final extension for 10 min at 72°C.

The specific amplification primers used for 16S rRNA molecular identification were as follows:
Forward primer 16S-F: 5'-AGAGTTTGATCCTGGCTCAG-3' (SEQ ID NO: 3);
Reverse primer 16S-R: 5'-TACGGCTACCTTGTTACGACTT-3' (SEQ ID NO: 4).

The amplified products were sent to Shanghai Sangon Biotech for sequencing.

Among them, the 16S sequence of the suspected *Akkermansia* sp. strain 1 (number N21169) obtained by sequencing was as shown in SEQ ID NO: 5. The 16S sequence of suspected *Akkermansia* sp. strain 2 (number N21116) was as shown in SEQ ID NO: 6. A NCBI BLAST^{®} tool was used for sequence search, it was found that their sequence similarities with the 16S rRNA of the standard strain of the *Akkermansia muciniphila* ATCC BAA-835 were 95.45% and 95.53% respectively. Thus, it can be seen that these two strains may be new undiscovered strains of the *Akkermansia* sp.

5'-
GCGGGCGGGTGGAAGACATGCAAGTCGAACGGAAGAATGCTAGCTTGCTAATATT
CTTCAGTGGCGCACGGGTGAGTAACACGTGAGCAACCTACCTTCGAGTGGGGGAT
AGCCCCGGGAAACTGGGATTAATACCGCATGAGGTCGAAAGATCAAAGCAGCAAT
GCGCTTGAAGATGGGCTCGCGTCCTATTAGTTAGTTGGTGAGGTAACGGCTCACCA
AGGCGATGACGGGTAGCCGGTCTGAGAGGATGTCCGGCCACACTGGAACTGAGA
CACGGTCCAGACACCTACGGGTGGCAGCAGTCGAGAATCATTCACAATGGGGGCA
ACCCTGATGGTGCGACGCCGCGTGGGGGAAGAAGGTCTTCGGATTGTAAACCCCTGT
CATGTGGGAGCAAGCAGCAATGTTGATAGTACCACAAGAGGAAGAGACGGCTAAC
TCTGTGCCAGCAGCCGCGGTAATACAGAGGTCTCAAGCGTTGTTCGGAATCACTG
GGCGTAAAGAGTACGTAGGCTGTTTTCTAAGTCAGGTTGAAAGGCAGGGGCTCAA
CCCCTGGACTGCACATGATACTGGGAGACTGGAGTAATGGAGGGGGAACCGGAAT
TCTCGGTGTAGCAGTGAAATGCGTAGATATCGAGAGGAACACTCGTGGCGAAGGC
GGGTTCCTGGACATTAACTGACGCTGAGGTACGAAGGCCAGGGTAGCGAAAGGG
ATTAGATACCCCTGTAGTCCTGGCAGTAAACGGTGCACGCTTGG
TGTGAGGGGACTCGACCCCCCTCGTGCCGGAGCTAAGCGTGCCGCCTGGGGAGTA
CGGTCGCAAGATTAAAACTCAAAGAAATTGACGGGGACCCGCACAAGCGGTGGA
GTATGTGGCTTAATTCGATGCAACGCGAAGAACCTTACCTGGGCTTGACATGTAAT
GAACAACATGTGAAAGCATGCGACTCTTCGGAGGCGTTACACAGGTGCTGCATGG
CCGTCGTCAGCTCGTGTCGTGAGATGTTTGGTTAAGTCCAGCAACGAGCGCAACC
CCTGTGGCCAGTTACCAGCACGTTATGGTGGGGACTCTGGCAAGACTGCCCAGAT
CAACTGGGAGGAAGGTGGGGACGACGTCAGGTCAGTATGGCCCTTATGCCCAGGG
CTGCACACGTACTACAATGCCCAGAACAGAGGGGGCCGAAGCCGTGAGGCGGAG
GAAATCCTGAAAACTGGGCCCAGTTCGGACTGTAGGCTGCAACCCGCCTACACGA
AGCCGGAATCGCTAGTAATGGCGCATCAGCTACGGCGCCGTGAATACGTTCCCGG
GTCTTGTACACACCGCCCGTCACATCATGGAAGCCGGTCGCACCCGAAGTATCTAG
AGCCAACCGCAAGGAGGCAGGGTCCC-3' (SEQ ID NO: 5).

5’-
GCGGGGCGCGTGCAAAAGACATGCAAGTCGAACGGAAGAATGCTAGCTTGCTAAT
ATTCTTCAGTGGCGCACGGGTGAGTAACACGTGAGCAACCTACCTTCGAGTGGGG
GATAGCCCCGGGAAACTGGGATTAATACCGCATGAGGTCGAAAGATCAAAGCAGC
AATGCGCTTGAAGATGGGCTCGCGTCCTATTAGTTAGTTGGTGAGGTAACGGCTCA
CCAAGGCGATGACGGGTAGCCGGTCTGAGAGGATGTCCGGCCACACTGGAACTGA
GACACGGTCCAGACACCTACGGGTGGCAGCAGTCGAGAATCATTCACAATGGGGG

CAACCCTGATGGTGCGACGCCGCGTGGGGGAAGAAGGTCTTCGGATTGTAAACCC
CTGTCATGTGGGAGCAAGCAGCAATGTTGATAGTACCACAAGAGGAAGAGACGGC
TAACTCTGTGCCAGCAGCCGCGGTAATACAGAGGTCTCAAGCGTTGTTCGGAATCA
CTGGGCGTAAAGAGTACGTAGGCTGTTTTCAAGTCAGGTGTGAAAGGCAGGGGCTC
AACCCCTGGACTGCACATGATACTGGGAGACTGGAGTAATGGAGGGGGAACCGGA
ATTCTCGGTGTAGCAGTGAAATGCGTAGATATCGAGAGGAACACTCGTGGCGAAG
GCGGGTTCCTGGACATTAACTGACGCTGAGGTACGAAGGCCAGGGTAGCGAAAGG
GATTAGATACCCCTGTAGTCCTGGCAGTAAACGGTGCACGCTTGGTGTGAGGGGAC
TCGACCCCCCTCGTGCCGGAGCTAACGCGTTAAGCGTGCCGCCTGGGGAGTACGG
TCGCAAGATTAAAACTCAAAGAAATTGACGGGGACCCGCACAAGCGGTGGAGTAT
GTGGCTTAATTCGATGCAACGCGAAGAACCTTACCTGGGCTTGACATGTAATGAAC
ATGTGAAAGCATGCGACTCTTCGGAGGCGTTACACAGGTGCTGCATGGCCGTCGT
CAGCTCGTGTCGTGAGATGTTTGGTTAAGTCCAGCAACGAGCGCAACCCCTGTGG
CCAGTTACCAGCACGTTATGGTGGGGACTCTGGCAAGACTGCCCAGATCAACTGG
GAGGAAGGTGGGGACGACGTCAGGTCAGTATGGCCCTTATGCCCAGGGCTGCACA
CGTACTACAATGCCCAGAACAGAGGGGGCCGAAGCCGTGAGGCGGAGGAAATCC
TGAAAACTGGGCCCAGTTCGGACTGTAGGCTGCAACCCGCCTACACGAAGCCGGA
ATCGCTAGTAATGGCGCATCAGCTACGGCGCCGTGAATACGTTCCCGGGTCTTGTACA
CACCGCCCGTCACATCATGGAAGCCGGTCGCACCCGAAGTATCTAGAGCCAACCG
CAAGGAGGCAGGGTCCTAAGGGT-3' (SEQ ID NO: 6).

In order to further determine the evolutionary relationship of the above-mentioned strains, the two suspected strains were subjected to whole genome sequencing, and the two suspected strains and only two members of the *Akkermansia* sp. were subjected to an ANI analysis.

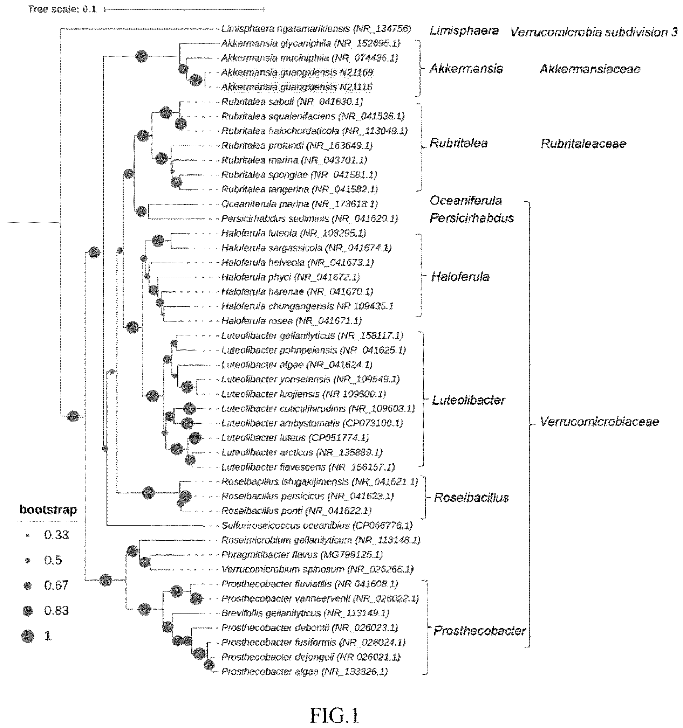

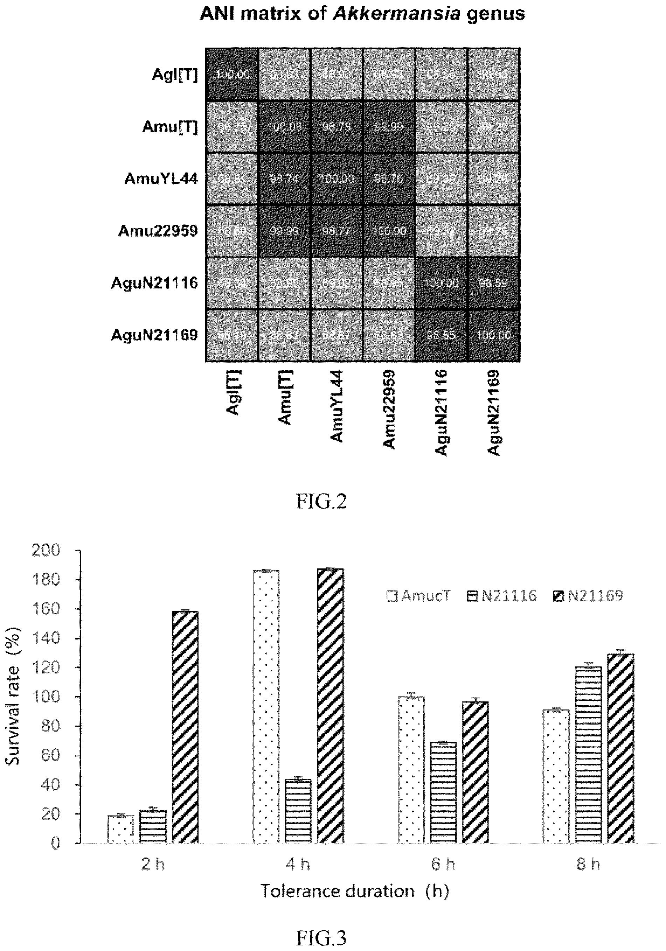

The results were as shown in FIGs. 1 and 2.

The results showed that the ANI values of N21116 and *Akkermansia muciniphila* ATCC BAA-835^{T} of *Akkermansia* sp. and *Akkermansia saccharophila* Py^{T} isolated from pythons were 68.95% and 68.34%, respectively. The ANI values of N21169 and *Akkermansia muciniphila* ATCC BAA-835^{T} of *Akkermansia* sp. and *Akkermansia saccharophila* Py^{T} isolated from pythons were 68.83% and 68.49% respectively, which met the ANI judging criteria (95%-96%) of new species. In addition, the sequence identity between N21116 and N21169 was 98.59%. It can be seen that N21116 and N21169 belong to the same strain.

Considering that the two strains of microorganisms were isolated from Guangxi, the inventor tentatively named them *Akkermansia guangxiensis,* and the Latin name was tentatively *Akkermansia guangxiensis* sp. nov.

The evolutionary tree based on the 16S rRNA sequences of the two strains of *Akkermansia guangxiensis* isolated in this example is as shown in FIG 1, which reveals the affiliation to Verrucomicrobiota, Verrucomicrobiae, Verrucomicrobiales, Akkermansiaceae and *Akkermansia* sp.

The two strains of *Akkermansia guangxiensis* (N21116 and N21169) obtained in the above examples were sent to the Guangdong Microbial Culture Collection Center (GDMCC) on December 2, 2022 for preservation at address of 5^{th} Floor, No.59 Building, No. 100, Xianlie Zhong Road, Guangzhou, China. The taxonomic names are *Akkermansia* sp., with the accession numbers of GDMCC No: 62888 and 62889.

### Levels resisting to artificial intestinal fluid of Akkermansia guangxiensis N21116 and N21169

The specific experimental steps were as follows: the *Akkermansia guangxiensis* N21116 and N21169 obtained in the above example were cultured and activated overnight (30°C-37°C, rotation speed 150 rpm-220 rpm), and the concentration of the bacterial solution was diluted to OD₆₀₀=1 with PBS. The bacterial solution was inoculated into the artificial intestinal fluid with pH=8 at an inoculation volume ratio of 10%. After 2 h, 4 h, 6 h, and 8 h, 100 µL of solution was taken and spread in the plate of solid culture medium with mucin. After culturing for 72 h at 37°C in an anaerobic workstation, the number of single colonies was counted, and the survival rates of *Akkermansia guangxiensis* N21116 and N21169 in the artificial intestinal fluid were calculated according to the number of single colonies.

Where the preparation method of the artificial intestinal fluid was as follows: 6.8 g of potassium dihydrogen phosphate was taken, 500 mL of water was added for dissolving the potassium dihydrogen phosphate, the pH value was adjusted to 8.0 with 0.1 mol/L of sodium hydroxide solution, then 10 g of trypsin was added and diluted with water to 1000 mL, then the artificial intestinal fluid was obtained.

At the same time, the standard strain of *Akkermansia muciniphila* ATCC BAA-835 served as control group.

The results were shown in FIG. 3.

It can be found that in a short period of time, the standard strain of *Akkermansia muciniphila* ATCC BAA-835 had better tolerance to the artificial intestinal fluid than *Akkermansia guangxiensis* N21116 and N21169. However, after processing for 8 h in the pH=8.0 artificial intestinal fluid, the survival rates of *Akkermansia guangxiensis* N21116 and N21169 were 120% and 129%, which were significantly higher than that of the standard strain of *Akkermansia muciniphila* ATCC BAA-835 (91%). This showed that *Akkermansia guangxiensis* N21116 and N21169 in the examples of the present invention had good tolerance to the artificial intestinal fluid, had the potential to colonize in the human gastrointestinal tract for a long time, and can meet the conditions for serving as probiotics.

### Safety evaluation of Akkermansia guangxiensis N21116 and N21169

In order to ensure the safety of *Akkermansia guangxiensis* N21116 and N21169 obtained in the above embodiments in various applications, the safety evaluation of N21116 and N21169 was carried out in this example.

Specific testing items included:
The *Akkermansia guangxiensis* N21116 and N21169 obtained in the above examples were subjected to whole genome sequencing to detect the presence of virulence factors and pathogenic genes. Under the conditions of sequence similarity ≥ 85% and e value < 10⁻⁵, the existing VFDB (Virulence Factor Database) virulence factor database was searched, and as a result, any virulence factors and pathogenic genes (see Table 2 for details) were not matched, therefore, it can be concluded from the genetic level that the *Akkermansia guangxiensis* N21116 and N21169 had no risk of pathogenicity.

**Table 2 Carrying status of virulence factors and pathogenic genes**

| Strain | sequence | Initial | Termination | Matching genes | Cover degree | Match situation | Notch | Coverage factor % | Similarity % | Gene number |
|---|---|---|---|---|---|---|---|---|---|---|
| N21116 | -- | -- | -- | -- | -- | -- | -- | -- | -- | -- |
| N21169 | -- | -- | -- | -- | -- | -- | -- | -- | -- | -- |

| | | | | | | | | | | |
|---|---|---|---|---|---|---|---|---|---|---|
| Note: "--" means null. | | | | | | | | | | |

### Antioxidant levels of Akkermansia guangxiensis N21116 and N21169

The *Akkermansia guangxiensis* N21116 and N21169 obtained in the above examples were placed in a synthetic liquid medium, after the anaerobic culture for 72 h at 37°C, the *Akkermansia guangxiensis* N21116 and N21169 were centrifuged for 10 min at 8000 rpm and at 4°C to separate the supernatant and cell pellet. The cell pellet was resuspended in PBS, and the cell concentration was adjusted to 1×10¹⁰ CFU/mL. After ultrasonic lysis, the *Akkermansia guangxiensis* N21116 and N21169 were centrifuged for 10 min at 8000 rpm and at 4°C, and the supernatant was collected to obtain cell-free extracts (CFE).

Hydroxyl radical, peroxide anion radical scavenging ability and SOD activity of cell-free extracts were detected (using hydroxyl radical test kits, glutathione peroxidase (GSH-PX) test kits and total superoxide dismutase (SOD) test kits from Nanjing Jiancheng Bioengineering Institute), referring to the instruction manual for the determination method.

In addition, the standard strain of *Akkermansia muciniphila* ATCC BAA-835 and *Akkermansia muciniphila* A21028 served as control groups.

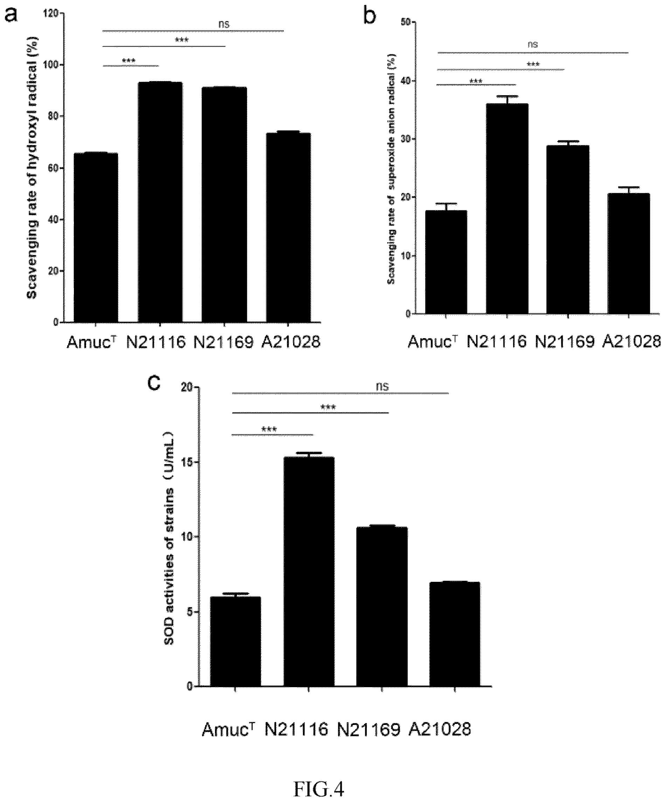

The results were as shown in FIG. 4.

It showed that *Akkermansia guangxiensis* N21116 was significantly better than the standard strain of *Akkermansia muciniphila* ATCC BAA-835 and *Akkermansia muciniphila* A21028 (P < 0.001) in three indicators: hydroxyl radical scavenging rate, peroxide anion scavenging rate and SOD activity, especially in terms of hydroxyl radical scavenging rate, the *Akkermansia guangxiensis* N21116 and N21169 both reach 90%, which was much higher than 65.56% of the standard strain and 73.38% of A21028, reflecting extremely excellent antioxidant capacity. Moreover, the *Akkermansia guangxiensis* N21169 was also better than the two kinds of *Akkermansia muciniphila.* Therefore, it can be judged that both *Akkermansia guangxiensis* N21116 and N21169 had high antioxidant capacity.

### Nematode fat-reducing test of Akkermansia guangxiensis N21116 and N21169

The *Akkermansia guangxiensis* N21116 and N21169 obtained in the above examples were cultured in a synthetic liquid culture medium, and *escherichia coli* OP50 was cultured in a LB liquid culture medium, with culture conditions of temperature 37°C, time 20h, rotation speed 200rpm. The *Akkermansia guangxiensis* N21116 and N21169 and *escherichia coli* OP50 bacterial solution (both bacterial solution concentrations were 10⁷ CFU/mL) were taken in respective to be coated on a nematode growth medium (NGM) and cultured overnight.

Where the NGM culture medium formula was as follows: 3 g sodium chloride, 17 g agar powder, 2.5 g peptone, and 975 mL deionized water. After the above ingredients were thoroughly mixed, the mixture was sealed with tin foil. After high-pressure steam sterilization for 20 min, the mixture was cooled to 55°C in a water bath. 1 mL of sterilized 1M CaCl₂, 1 mL of 1M MgSO₄, 25 mL of 1M KPO₄ buffer, and 1 mL of 5 mg/mL cholesterol (dissolved in 95% ethanol) were added under sterile conditions.

After being centrifuged, the thawed nematodes (*Caenorhabditis elegans*) were added to NGM mediums with OP50 in respective and placed in a 20°C incubator to be cultured for 3 days.

The nematodes used in experiments were subjected to synchronization treatment: the cultured nematodes were suspended with a M9 buffer, aspirated into culture tubes, a lysis solution (5N NaOH by mass and 5% sodium hypochlorite solution by volume) was added to each tube, lysed for 6 min, and centrifuged for 1 min at 3500 r/min. The supernatant was discarded to be respectively washed for 4 times with M9 buffer. Centrifuging was carried out again and the supernatant was discarded. The precipitate was transferred to the NGM culture medium to be cultured overnight at a constant temperature of 20°C to obtain L 1-stage nematode larvae. After washing and centrifuging once with the M9 buffer, the nematode larvae was transferred to the NGM plate containing OP50 and cultured for 28-30 h at 20°C to obtain synchronized nematodes, namely L4-stage nematodes.

The obtained synchronized nematodes were randomly divided into three groups: a blank group (OP50), a N21116 group and a N21169 group, with 150 worms in each group. The day when L4-stage nematodes were picked was recorded as day 0. During the experiment, the NGM medium containing OP50 and *Akkermansia guangxiensis* N21116 and N21169 for the nematodes was replaced every 2 days. After washing three times with chilled M9 buffer on day 7, the nematodes were resuspended in 4% paraformaldehyde to be shaken slightly for 1 h at a room temperature, centrifuged for 1 min at 3000-4000 rpm to remove the supernatant and wash twice with the M9 buffer. Subsequently, the nematodes were resuspended in a mixed PBS solution containing 60% (v/v) isopropyl alcohol and 0.01% (v/v) Triton X-100, and incubated for 15 min. After the nematodes settled, the isopropyl alcohol was removed, 1 mL of 40% oil red O stain was added, to be incubated on a 25°C shaker for 1-2 h and fully stained. The dye was removed, the washing was performed twice with the M9 buffer. 200 µL of M9 buffer was added, and the nematodes were photographed one by one under an inverted fluorescence microscope. Image J was used to count the light intensity data of the stained area of the image.

In addition, the standard strain of *Akkermansia muciniphila* ATCC BAA-835 and *Akkermansia muciniphila* A21028 served as control groups.

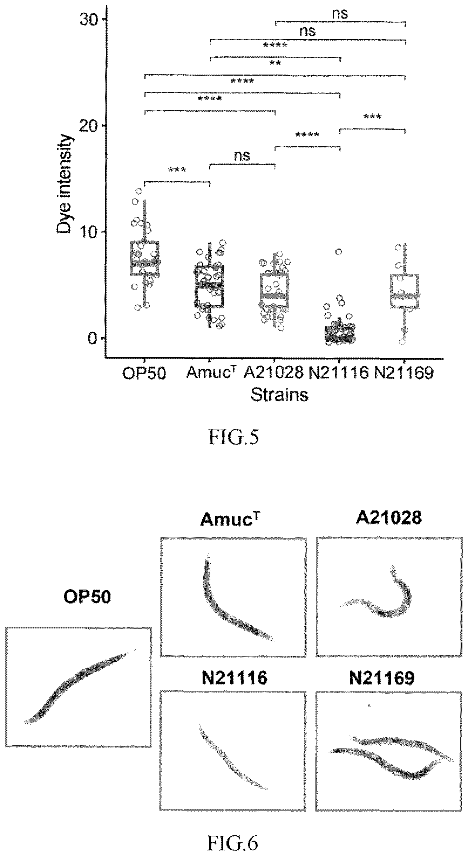

The results were as shown in FIGs. 5 and 6.

It can be found that the inhibition rate of fat particle formation in nematodes fed with the standard strain of *Akkermansia muciniphila* ATCC BAA-835 is 33.9%. The inhibition rates of fat particle formation in nematodes fed with *Akkermansia muciniphila* A21028 and *Akkermansia guangxiensis* N21169 were 42.1% and 44.8%, respectively, which were not significantly different from the standard strain group (P > 0.05). However, the inhibition rate of fat particle formation in the *Akkermansia guangxiensis* N21116 feeding group reached 89.0%, which significantly inhibiting more than the above three groups by 55.1% (P < 0.01), 46.9% (P < 0.0001) and 44.2% (P < 0.001). Moreover, from the nematode fat staining micrograph (FIG. 4), it can be intuitively seen that the nematode fat particles in the *Akkermansia guangxiensis* N21116 feeding group were sparsely distributed, smaller in the particle size, and more slender in nematode body. It can be seen that *Akkermansia guangxiensis* N21116 had a significant fat-lowering effect.

### Inhibition of Akkermansia guangxiensis N21116 and N21169 to proliferation of colon cancer cells

The human colon cancer cell line HCT116 was cultured in RPMI1640 (TransGen) culture medium containing 10% fetal bovine serum. When the cells grew to the logarithmic phase, they were digested with trypsin and counted by a hemocytometer. Cells were evenly inoculated in a 96-well plate at 5 × 10³ cells/well and cultured overnight in a cell culture incubator containing 5% CO₂ at 37°C. The activated *Akkermansia guangxiensis* N21116 or N21169 were centrifuged to prepare a bacterial suspension with a bacterial concentration of 1×10⁸ CFU/mL. A part of the bacterial suspension was taken to be centrifuged to obtain bacterial cells, washing was carried out twice with PBS, and the bacterial suspension was inactivated in a 70°C water bath for 30 min to obtain inactivated bacteria. By grouping: blank group (adding equal amount of PBS), N21116 live bacteria group, N21169 live bacteria group, N21116 inactivated bacteria group, N21169 inactivated bacteria group, six repetitions for each group. After incubating with the cells for 72 h, a CCK-8 reagent (Solarbio) was used to detect the proliferation of HCT116 cells (operate according to the instructions for use).

In addition, the standard strain of *Akkermansia muciniphila* ATCC BAA-835 served as a control group.

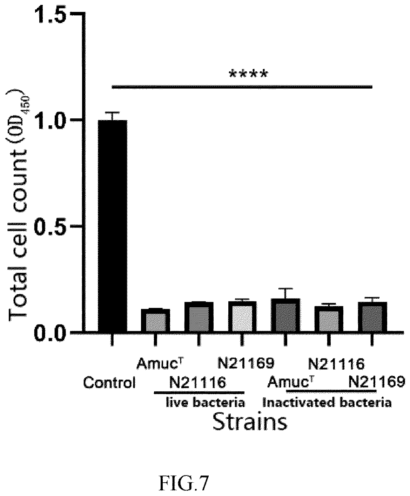

The results were as shown in FIG. 7.

It can be found that *Akkermansia guangxiensis* N21116 and N21169 inhibited the growth of human colon cancer cell line HCT116 by 85%. The live bacteria group and the inactivated bacteria group were basically the same (P > 0.05), and not lower than those of the standard strain of *Akkermansia muciniphila* ATCC BAA-835 (P > 0.05). It can be seen that the live and inactivated bacterial forms of *Akkermansia guangxiensis* N21116 and N21169 had a growth inhibition rate of no less than the standard strain of the human colon cancer cell line HCT116, and had significant ability to inhibit the growth of human colon cancer cells.

### Products capable of resisting oxidation, inhibiting tumor and reducing fat based on Akkermansia guangxiensis N21116 or N21169

The *Akkermansia guangxiensis* N21116 or N21169 strain was taken for fermentation and propagation, and then the fermented and propagated bacterial solution was centrifuged and freeze-dried to obtain freeze-dried powder. Then compounding was carried out according to the following formula to obtain a food additive or dietary supplement containing the *Akkermansia guangxiensis* N21116 or N21169.

Based on the human body dosage and calculated based on weight percentage, the formula of food additives or dietary supplements containing *Akkermansia guangxiensis* N21116 or N21169 was: 10% *Akkermansia guangxiensis* N21116 or N21169 freeze-dried powder, 15% tea polyphenol (purchased from Thankcome Biological Science and Technology (SuZhou) Co., Ltd.), 15% bayberry anthocyanin extract (purchased from Thankcome Biological Science and Technology (SuZhou) Co., Ltd.), 50% maltodextrin and 10% soluble dietary fiber (purchased from Thankcome Biological Science and Technology (SuZhou) Co., Ltd.). The preparation method was: obtaining the product after thoroughly mixing the above raw materials.

In summary, the above test results showed that, among the existing *Akkermansia* sp., only *Akkermansia guangxiensis* N21116 and N21169 had excellent functions of resisting oxidation, losing weight, reducing fat and inhibiting tumor growth, and can survive in the artificial intestinal fluid environment, therefore, it can be shown that it has the potential to be colonized in the gastrointestinal tract of the body, to stably exert the effects resisting oxidation, losing weight, reducing fat and inhibiting tumor growth for long-term.

The above examples are preferred embodiments of the present invention, but the embodiments of the present invention are not limited to the above examples. Any other changes, modifications, substitutions, combinations and simplifications made without departing from the spirit and principles of the present invention should be equivalent substitutions, and are all included in the protection scope of the present invention.

## Claims

1. A product containing *Akkermansia muciniphila,* comprising food, food additives, feed, feed additives, medicines and cosmetics;
wherein the *Akkermansia muciniphila* is *Akkermansia guangxiensis* N21116 and/or *Akkermansia guangxiensis* N21169;
wherein the *Akkermansia guangxiensis* N21116 has a taxonomic name of *Akkermansia* sp., deposited in the Guangdong Microbial Culture Collection Center on December 2, 2022, with an accession number of GDMCC No: 62888; and
wherein the *Akkermansia guangxiensis* N21169 has a taxonomic name of *Akkermansia* sp., deposited in the Guangdong Microbial Culture Collection Center on December 2, 2022, with an accession number of GDMCC No: 62889.

2. The product according to claim 1, wherein a mass fraction of the *Akkermansia muciniphila* in the product is greater than or equal to 1%, preferably greater than or equal to 8%, and more preferably greater than or equal to 10%.

3. The product according to claim 1, wherein the product further comprises other excipients, wherein the excipients comprise pharmaceutically acceptable adjuvants, food additives, and cosmetic accessories.

4. A food additive containing *Akkermansia muciniphila,* wherein the food additive comprises freeze-dried powder of the *Akkermansia guangxiensis* N21116 and/or the *Akkermansia guangxiensis* N21169, plant extracts, maltodextrin and dietary fiber.

5. The food additive according to claim 4, wherein in terms of mass percentage, the food additive comprises 5-15% freeze-dried powder of *Akkermansia guangxiensis* N21116 and/or *Akkermansia guangxiensis* N21169, 15-40% plant extracts, 40-60% maltodextrin and 5-15% dietary fiber.

6. The food additive according to claim 4 or 5, wherein the plant extracts comprise tea polyphenol and bayberry anthocyanin extracts.

7. Use of *Akkermansia muciniphila* in the preparation of food, food additives, feeds, feed additives, medicines and cosmetics; and the *Akkermansia muciniphila* is *Akkermansia guangxiensis* N21116 and/or *Akkermansia guangxiensis* N21169.

8. The use according to claim 7, wherein the food, food additives, feeds, feed additives, medicines and cosmetics have at least one of the following functions (1) to (3):
(1) resisting oxidation;
(2) reducing fat; and
(3) inhibiting tumor proliferation.

9. The use according to claim 8, wherein the tumor is gastrointestinal tumor.

10. A method of preventing and/or treating tumors, comprising: administering a therapeutically effective amount of *Akkermansia muciniphila* to a tumor patient, wherein the *Akkermansia muciniphila* is *Akkermansia guangxiensis* N21116 and/or *Akkermansia guangxiensis* N21169; and
the tumor is preferably gastrointestinal tumor.
